(19) **Europäisches Patentamt**

**European Patent Office**

**Office européen des brevets**

(11) Numéro de publication: **0 314 537**

**A1**

(12) **DEMANDE DE BREVET EUROPEEN**

(21) Numéro de dépôt: **88402509.9**

(22) Date de dépôt: **04.10.88**

(51) Int. Cl.⁴: **C 12 M 1/34**

(30) Priorité: **30.10.87 FR 8715114**

(43) Date de publication de la demande:
**03.05.89 Bulletin 89/18**

(84) Etats contractants désignés: **DE GB IT**

(71) Demandeur: **JEULIN**
**28 rue Lavoisier Zone Industrielle no. 2 B.P. 3110**
**F-27031 Evreux Cédex (FR)**

(72) Inventeur: **Videaud, André**
**3 rue des Bruyères**
**F-87270 Couzeix (FR)**

(74) Mandataire: **Fruchard, Guy et al**
**CABINET BOETTCHER 23, rue la Boétie**
**F-75008 Paris (FR)**

(54) Dispositif de mesure de l'activité cellulaire.

㉟ Le dispositif de mesure d'activité cellulaire selon l'invention comporte une enceinte tubulaire cylindrique 1 sur laquelle est fixée une sonde d'analyse de gaz 13 et un bouchon cylindrique 2 ayant une section semblable à l'enceinte tubulaire et monté pour coulisser dans cette enceinte.

Fig. 1

## Description

### Dispositif de mesure de l activité cellulaire.

La présente invention concerne un dispositif de mesure de l'activité cellulaire liée au métabolisme cellulaire : respiration, photosynthèse, réactions enzymatiques.

On sait que les cellules sont des organismes vivants et que leur degré d'activité peut être mesuré en mesurant les variations du taux d'oxygène, ou d'un autre paramètre comme le PH, du milieu dans lequel elles sont contenues. Une mesure de ce type est utilisée en particulier pour contrôler le processus de fabrication de la bière. Toutefois, une telle variation est très faible à l'échelon unitaire et il n'existe pas à ce jour de dispositif permettant de visualiser convelablement le phénomène lorsque celui-ci est exposé à des étudiants.

Un but de la présente invention est de proposer un dispositif de mesure de l'activité cellulaire ayant une structure appropriée à une bonne illustration du phénomène pour des étudiants.

En vue de la réalisation de ce but, on propose selon l'invention un dispositif de mesure de l'activité cellulaire comportant une enceinte tubulaire cylindrique sur laquelle est fixée une sonde d'analyse et un bouchon cylindrique ayant une section semblable à l'enceinte tubulaire et monté pour coulisser dans cette enceinte. Ainsi, le volume interne de l'enceinte est aisément adapté au volume des cellules contenues dans l'enceinte de sorte que la sonde réagit très rapidement aux variations du paramètre mesuré dans le milieu contenant les cellules.

Selon une version avantageuse de l'invention, le dispositif comporte un agitateur monté dans l'enceinte et ayant une tige traversant le bouchon. Ainsi le milieu contenant les cellules est aisément homogénéisé et on obtient donc une meilleure information en provenance de la sonde.

Selon un autre aspect avantgeux de l'invention, le bouchon comporte une face interne conique ayant un sommet coïncidant avec la tige de l'agitateur. Ainsi on évite d'emprisonner des bulles gazeuses à l'intérieur de l'enceinte et on obtient donc une mesure encore améliorée en particulier dans le cas où le phénomène mesuré est le taux d'oxygène.

Selon encore un autre aspect avantageux de l'invention, le dispositif comporte un canal de faible section traversant le bouchon. Ainsi on évite d'une part une augmentation de pression à l'intérieur de l'enceinte lors d'un dégagement gazeux important et on permet l'introduction de substances modifiant le métabolisme cellulaire tout en effectuant une détection continue du phénomène mesuré.

Selon un mode de réalisation préféré de l'invention, la sonde d'analyse est montée à une extrémité de l'enceinte tubulaire opposée au bouchon. Ainsi, on minimise les perturbations de mesure résultant des échanges gazeux avec l'extérieur autour du bouchon ou à travers celui-ci.

Selon encore un autre aspect avantageux de l'invention, l'enceinte tubulaire comporte une paroi transparente. Ainsi, on observe également visuellement le déroulement de la réaction et on peut soumettre le milieu cellulaire à des radiations, notamment de la lumière.

D'autres caractéristiques et avantages de l'invention ressortiront encore à la lecture de la description qui suit en liaison avec les figures ci-jointes qui illustrent un mode de réalisation particulier non limitatif de l'invention et parmi lesquelles :

- la figure 1 est une vue en perspective du dispositif selon l'invention,
- la figure 2 est une vue en coupe axiale de l'enceinte tubulaire.

En référence aux figures, le dispositif de mesure d'activité cellulaire selon l'invention comporte une enceinte tubulaire cylindrique de section circulaire 1 en matière plastique transparente à une extrémité de laquelle est engagé un bouchon 2, également en matière plastique, cylindrique ayant une section très voisine de la section interne de l'enceinte tubulaire 1 et monté pour coulisser directement dans cette enceinte.

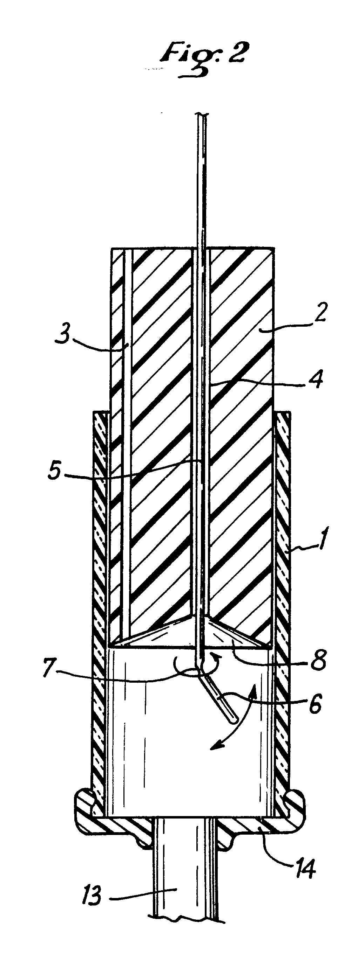

Le bouchon 2 est traversé par un canal 3 de faible section s'étendant parallèlement à l'axe du bouchon. Le bouchon 2 comporte également un canal 4 dans lequel s'étend la tige d'un agitateur formé d'un fil de nylon 5 comportant une partie 6 s'étendant en saillie à l'intérieur (fig. 2) de l'enceinte lorsque le bouchon est mis en place, et une zone amincie 7 au voisinage d'une face interne 8 du bouchon 2.

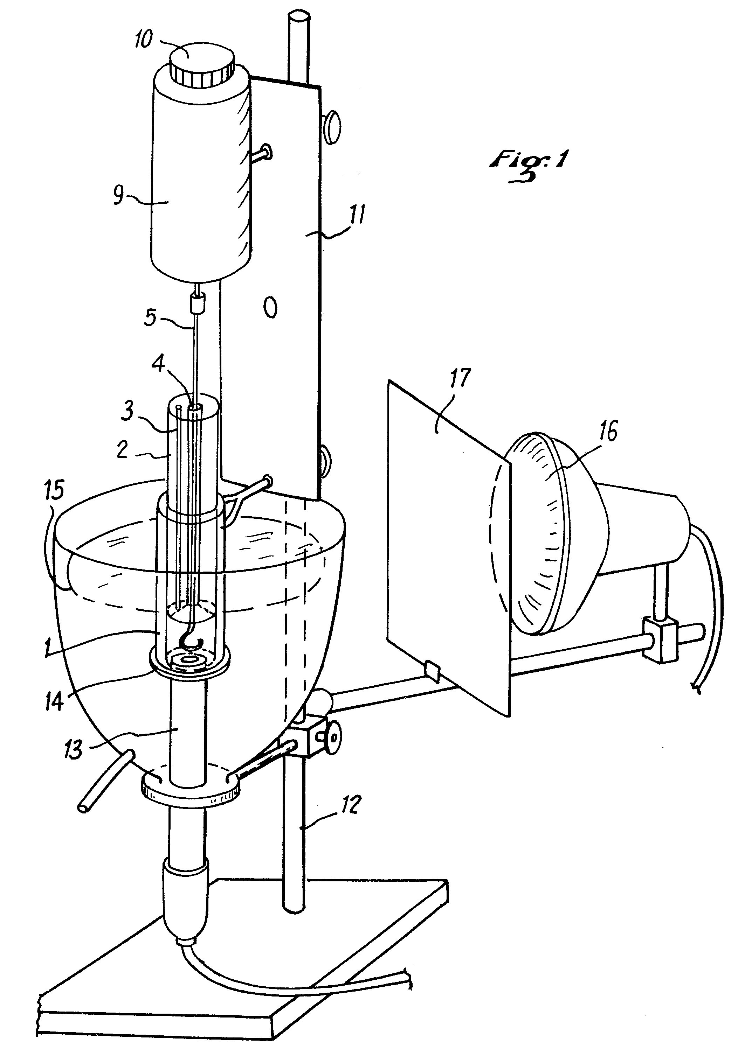

D'autres formes de réalisation sont possibles, par exemple dans le cas de la figure 1 l'agitateur est formé d'un fil d'acier inoxydable dont la partie inférieure est recourbée pour former une boucle ouverte réalisant une palette de brassage.

A son extrémité débouchant à l'extérieur du bouchon, le fil 5 est raccordé à un moteur électrique 9, par exemple un petit moteur à pile dont la vitesse de rotation est commandée par un bouton potentiométrique 10. L'enceinte tubulaire 1 et le moteur 9 sont par exemple fixés à un support 11 monté de façon coulissante sur une potence 12.

La face interne 8 du bouchon 2 est conique et le sommet de la partie conique coïncide avec la tige 5 de l'agitateur; ainsi un dégagement gazeux s'échappe le long de la tige 5 de l'agitateur.

A une extrémité de l'enceinte 1 opposée au bouchon 2, le dispositif comporte une sonde d'analyse 13, par exemple une sonde oxymétrique, montée de préférence de façon amovible sur une capsule étanche 14, par exemple une capsule en matière plastique semi-rigide.

Le moteur électrique 9 est de préférence monté à une distance suffisante de la sonde 13 pour ne pas perturber la mesure effectuée par celle-ci.

Dans le mode de réalisation préféré illustré, le dispositif comporte en outre une enceinte thermostatique 15, ici un bol en matière plastique transparente entourant la partie inférieure de l'enceinte tubulaire 1 au niveau où celle-ci contient le milieu cellulaire. Le bol 15 est par exemple rempli avec de l'eau à la température désirée assurant un volant thermique important et tendant ainsi à maintenir le milieu cellulaire à une température sensiblement constante quel que soit le type de réaction s'y déroulant.

Lors de l'utilisation, le bouchon est retiré et les cellules dont on souhaite mesurer l'activité, par exemple des levures, des chloroplastes végétaux, des mitochondries ou d'autres cellules dont on souhaite étudier la physiologie, sont introduites dans l'enceinte dans un milieu liquide. L'enceinte est alors refermée au moyen du bouchon qui est de préférence dimensionné pour coulisser dans l'enceinte 1 avec un léger frottement permettant de maintenir le bouchon en place dans l'enceinte sans autre moyen de fixation.

Le bouchon est enfoncé dans l'enceinte 1 de préférence jusqu'à ce que le liquide atteigne le sommet de la face conique 8.

La sonde d'analyse 13, convenablement reliée à un appareil de mesure, donne alors une indication de l'activité des cellules. Pour montrer les variations d'activité des cellules, on peut également introduire par le canal 3 des petites quantités de substance destinées à modifier le métabolisme cellulaire. Par exemple, dans le cas de levures, on peut introduire de l'eau sucrée pour activer la respiration ou au contraire un anesthésique pour ralentir la respiration, ces variations d'activités étant immédiatement détectées par la sonde d'analyse 13.

Il est également possible d'étudier la respiration sur des mitochondries isolées et l'activité catalasique due aux peroxytomes. Dans le cas de chloroplastes végétaux, on peut également mesurer les variations de l'activité de photosynthèse lorsque ceux-ci sont soumis à différentes radiations émises par exemple par une lampe 16 montée sur la potence 12 pour éclairer le milieu cellulaire éventuellement à travers un écran filtrant 17.

Bien entendu l'invention n'est pas limitée au mode de réalisation décrit et on peut y apporter des variantes de réalisation sans sortir du cadre de l'invention.

En particulier, si l'on souhaite poser le dispositif sur une table, on peut prévoir de fixer la sonde d'analyse de gaz sur la paroi latérale de l'enceinte à la base de celle-ci.

Par ailleurs, bien que l'on ait plus particulièrement envisagé une application pédagogique du dispositif selon l'invention pour illustrer auprès des étudiants l'activité des cellules, on peut également utiliser le dispositif selon l'invention à titre expérimental en laboratoire pour tester les réactions des cellules à certains milieux, le dispositif selon l'invention présentant l'avantage d'avoir des dimensions faibles et permettant ainsi l'analyse des réactions de petites quantités de cellules.

**Revendications**

1. Dispositif de mesure de l'activité cellulaire caractérisé en ce qu'il comporte une enceinte tubulaire cylindrique (1) sur laquelle est fixée une sonde d'analyse (13), et un bouchon cylindrique (2) ayant une section semblable à l'enceinte tubulaire et monté pour coulisser dans cette enceinte.

2. Dispositif de mesure de l'activité cellulaire selon la revendication 1, caractérisé en ce qu'il comporte un agitateur (6) monté dans l'enceinte et ayant une tige (5) traversant le bouchon.

3. Dispositif de mesure de l'activité cellulaire selon la revendication 2, caractérisé en ce que le bouchon comporte une face interne conique (8) ayant un sommet coïncidant avec la tige (5) de l'agitateur.

4. Dispositif de mesure de l'activité cellulaire selon la revendication 2 ou la revendication 3, caractérisé en ce que l'agitateur est formé d'un fil en matière plastique (5) comportant une zone amincie (7) au voisinage d'une extrémité interne (8) du bouchon.

5. Dispositif de mesure de l'activité cellulaire selon l'une des revendications 2 à 4, caractérisé en ce que l'agitateur est entraîné par un moteur électrique (9) à vitesse variable.

6. Dispositif de mesure de l'activité cellulaire selon l'une des revendications 1 à 5, caractérisé en ce qu'il comporte un canal (3) de faible section traversant le bouchon.

7. Dispositif de mesure de l'activité cellulaire selon l'une quelconque des revendications 1 à 6, caractérisé en ce que la sonde d'analyse (13) est montée à une extrémité de l'enceinte tubulaire opposée au bouchon (2).

8. Dispositif de mesure de l'activité cellulaire selon l'une quelconque des revendications 1 à 7, caractérisé en ce que l'enceinte tubulaire (1) comporte une paroi transparente.

9. Dispositif de mesure de l'activité cellulaire selon l'une des revendications 1 à 8, caractérisé en ce qu'il comporte une enceinte thermostatique entourant au moins partiellement l'enceinte tubulaire cylindrique.

10. Dispositif de mesure de l'activité cellulaire selon la revendication 9 prise dans son rattachement à la revendication 8, caractérisé en ce que l'enceinte thermostatique comporte une paroi transparente.

*Fig:1*

# Fig. 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | JOURNAL OF SCIENTIFIC INSTRUMENTS (JOURNAL OF PHYSICS E), vol. 1, no. 8, août 1968, pages 861-863; K.J. ADAMS: "An automatic recording cell counter for monitoring growing populations of unicellular micro-organisms" * Figure 1; résumé * | 1,5,8-10 | C 12 M   1/34 |
| A | EP-A-0 028 793  (KYOWA HAKKO KOGYO) * Figure 1; résumé; revendications 1-3 * | 1,5 | |
| A | FR-A-2 372 229  (HAUS DER GESUNDHEIT) * Revendications * | 1 | |

DOMAINES TECHNIQUES
RECHERCHES (Int. Cl.4)

C 12 M
G 09 B
C 12 K

Le présent rapport a été établi pour toutes les revendications

| Lieu de la recherche | Date d'achèvement de la recherche | Examinateur |
|---|---|---|
| LA HAYE | 30-01-1989 | EPAILLARD P.J.H.M. |